# EUROPEAN PATENT APPLICATION

(11) **EP 1 271 151 A1**
(43) Date of publication of application: **02.01.2003**
(21) Application number: 01115007.5
(22) Date of filing: 20.06.2001
(51) Int. Cl.: G01N 33/569

(54) **Assay and process for the detection of HPV antibodies**

(71) Applicant: Virofem Diagnostika GmbH, 56379 Holzappel (DE)
(72) Inventor: Hilfrich, Ralf, Dr., 65597 Hünfelden (DE); Sapp, Martin, Dr., 55278 Undenheim (DE)
(74) Representative: Grünecker, Kinkeldey, Stockmair & Schwanhäusser Anwaltssozietät

(57) **Abstract**

The invention relates to an assay and a process for the detection of HPV type-specific antibodies. In particular, the invention relates to an assay, comprising a substrate coated with a negatively charged macromolecule and at least one HPV type-restricted epitope, and a process for the detection of HPV antibodies using the assay of the invention.

## Description

The invention relates to an assay and a process for the detection of HPV antibodies.

Papillomaviruses are highly species- and tissue-specific viruses found primarily in higher vertebrates. They exclusively infect basal cells of epithelia and induce squamous epithelial and fibroepithelial tumors, e. g. warts (papillomas) and condylomata.

To date more than a hundred different human papillomaviruses (HPV) have been identified, as described in zur-Hausen, 1994. Although their genetic organization is very similar, they differ in their tissue specificity and the associated disease. Some HPV types are mainly restricted to the skin inducing benign (e.g. HPV-1, -2) or malignant lesions (e.g. HPV-5, -8), others induce intraepithelial lesions of the anogenital tract which are also classified as benign (e.g. HPV-6, -11) or malignant (e.g. HPV-16, -18, -31, -33 and others).

Papillomaviruses and the genetically unrelated polyomaviruses are grouped together into the family Papovaviridae because they share a number of structural features: a circular double-stranded DNA genome, associated with histones to form a minichromosome, and a spherical capsid of T=7 icosahedral symmetry. HPV capsids consist of 72 capsomeres, each a pentamer of the major capsid protein L1, as described in Baker, 1991 and Salunke, 1986 . 60 capsomeres are hexavalent, i.e. are surrounded by six other capsomeres, whereas 12 capsomeres are pentavalent. In addition, papillomavirus capsids probably contain twelve copies of the minor capsid protein L2 located at the center of the twelve pentavalent capsomeres, as described in Trus, 1997.

Only few HPVs have been purified in quantities sufficient for structural analysis due to the low virus content of many lesions and to the incapacity to develop tissue culture systems for large-scale propagation of papillomaviruses. Recently, however, virus-like particles (VLPs) have been produced by using recombinant vaccinia viruses, as described in Hagensee, 1993, Zhou, 1991 , baculoviruses, as described in Kirnbauer, 1992 , Rose, 1993 and Volpers, 1994 , or yeast, as described in Sasawaga, 1995 , to synthesize the major capsid protein L1 either alone or together with the minor capsid protein L2. VLPs do not contain DNA but are otherwise indistinguishable from virions according to cryoelectron microscopy and three-dimensional image reconstruction at 3.5-nm resolution, as described in Hagensee, 1994 . Since VLPs are amenable to genetic analysis, they offer a promising approach to unravel the still unknown mechanisms of assembly, cellular uptake, and disassembly of papillomaviruses.

VLPs have been shown to contain immunodominant HPV type-restricted epitopes and allow the detection of type-specific antibodies in the blood of infected individuals. No cross-reactivity has been reported so far. They have been used in a vast and still increasing number of epidemiological studies (Stanley, M., 1997) Two different formats for the use of VLP-based ELISAs have been reported so far.

VLPs were either coupled directly to surfaces like a microtiter plate or were coupled via conformation-dependent type-specific monoclonal antibodies that served as bridge. A problem associated with the use of microtiter plate with VLPs directly bonded thereto is that VLPs contain a fraction of incorrectly folded protein which will be detected therewith rendering the system unspecific with regard to the detection of HPV antibodies. In order to overcome this problem, conformation-dependent type-specific monoclonal antibodies were used to couple the VLPs to microtiter plates. However, this procedure is expensive and does not allow testing of several HPV-types at the same time because of the type specificity of the monoclonal antibodies.

Therefore, the problem underlying the present invention was to provide a reliable and inexpensive assay and a process for detection of HPV type-specific antibodies solving the problems associated with known assays and processes.

The solution of these problems is an assay for the detection of HPV antibodies comprising
a) a substrate coated with a negatively charged macromolecule, and
b) at least one type of virus-like particles (VLPs) or subunits of these virus like particles (VLPs) containing at least one HPV type-restricted epitope.

The substrate can be any known carrier capable to be used in an immunoassay, preferably selected from the group of microtiter plates or membranes. The material of the substrate may be any material to which a negatively charged macromolecule will bind.

Preferably, the substrate is selected from the group consisting of glass, polystyrene, nitrocellulose, nylon, polypropylene and polyethylene.

The substrate is coated with a negatively charged macromolecule. The negatively charged macromolecule is preferably a negatively charged polysaccharide selected from the group consisting of homo- and heteroglycans containing COO⁻ and/or SO₃⁻-groups, in particular sulphated-glucosaminoglycans e.g. Heparin. The negatively charged macromolecule, in particular Heparin, may be coupled to a carrier protein e.g. bovine serum albumin (BSA), or any other molecule that is capable of coupling the sulphated-glucosaminoglycans to the substrate.

The assay of the invention further comprises at least one type of virus-like particles (VLPs) or subunits of these virus like particles (VLPs) containing at least one HPV type-restricted epitope, preferably an VLP of HPV 6, 11, 16, 18, 31, 33, 35, 39, 45, 56, 58 or mixtures thereof or subunits of these VLPs. The HPV type-specific VLP contains at least one HPV type-restricted epitope. This epitope is preferably selected from the group consisting of at least one capsid protein selected from the group, consisting of L1 alone or L1 and L2 of HPV or parts of these proteins carrying HPV type-specific epitopes.

The at least one HPV type-restricted epitope will be bound to the substrate via the negatively charged macromolecule, preferably Heparin. In a preferred embodiment, a substrate comprising various wells is used and one HPV type-specific VLP is used in each well. However, also mixtures of HPV type-specific VLPs can be used in each well. Which embodiment is preferred depends on the intended use. Should it be of interest to detect one HPV type-specific antibody with the assay of the invention, it is preferred to use only one HPV type-specific VLP in each well. Should the intention be to rapidly determine whether more than one type-specific HPV antibody is present, it is preferred to use a mixture of HPV type-specific epitopes preferably a mixture of VLPs or subunits of these VLPs or VLPs or subunits of VLPs carrying more than one HPV type-specific epitopes in each well.

The assay of the invention may be prepared by selecting a substrate, contacting the substrate with the negatively charged macromolecule, thereby coating the substrate with the negatively charged macromolecule. Optionally, the coated substrate may be washed with water and/or PBS or any other appropriate buffer in order to remove any unbound material. The coated substrate may be stored under appropriate conditions, such as cooled or sealed. Under appropriate conditions the coated substrate is stable for at least 6 months without any loss of activity. Thus, the coated substrate can be prepared and stored until the assay should be used or shipped to the intended place where the assay will finally be prepared and used.

The substrate coated with the negatively charged macromolecule will subsequently be contacted with the at least one type of VLP or subunit thereof containing at least one HPV type-restricted epitope. The at least one type of VLP or subunit thereof is preferably contained in a solution, in particular a solution in PBS supplemented with high concentrations of salt preferably sodium chloride or cesium chloride (<2.4 M) or any other appropriate buffer. The assay may additionally be washed with the same solvent as used for the contacting solution containing the at least one type of VLP containing an HPV type-restricted epitope. Thereby an assay of the invention is obtained.

The assay of the invention is stable for at least 6 month. The assay may also be used directly in the process of the invention for detection of HPV antibodies.

Another solution of the problem underlying the invention is a process for the detection of HPV antibodies comprises the steps of
a) providing an assay of the invention
b) contacting the assay with a probe, and
c) detecting whether an HPV type-specific antibody is bound to the assay.

In step a) an assay according to the invention is provided. The assay is contacted in step b) with any probe in which an HPV type-specific antibody should be detected, such as a human serum, blood or any other liquid containing antibodies. In step c) of the process of the invention it is detected whether an HPV type-specific antibody is bound to the assay using an anti-human specific immunglobulin and any appropriate detection system, e.g. peroxidase/tetramethylbenzidine (TMB).

The advantage of the assay and the process of the invention as compared to assays or processes in which VLPs containing an HPV type-specific epitope is directly coupled to the substrate is (i) a faster kinetic of binding, (ii) only conformationally intact VLPs bind to the plate, and (iii) contaminating proteins from the cells used for synthesis will not bind to the plates. That means that denatured antigen is not retained on the microtiter plate. Since it is well established that linear epitopes of L1 protein induce a cross-reactive immune response, the detection of these cross-reactive antibodies is excluded, thus ensuring that the assay exclusively allows the detection of type-specific antibodies.

The advantage of the assay and the process of the invention as compared to the use of monoclonal antibodies as coupling components of the substrate and the HPV type-specific epitope, such as an VLP, is that VLPs of all types tested bind with similar affinities to the negatively charged macromolecule, in particular Heparin-BSA, whereas each VLP type requires its own conformation-dependent monoclonal antibody, since it was shown that these are type-specific. Thus, with the use of negatively charged macromolecules, such as sulphated-glucosaminoglycans (e.g. Heparin-BSA), as coupling components of the substrate and at least one type of VLP containing an HPV type-specific epitope a test can be designed that allows testing of several HPV-types in a single plate. This can either be done in one well for several HPV-types for screening purposes or in individual wells for each HPV type for HPV typing.

The invention is further illustrated with the following examples.

### Example 1

MaxiSorb microtiter plates (Nunc) were coated with 100 µl/well of 1 µg/ml Heparin-BSA (heparin covalently attached to bovine serum albumin; Sigma Aldrich) dissolved in phosphate buffered saline (PBS) overnight at 4°C. Plates were washed with PBS-0.05% Tween20 three times and subsequently incubated with blocking solution (PBS-0.05% Tween20-0.5% BSA) for 3 h at room temperature. Plates were washed again with PBS and H₂O and subsequently dried under vacuum overnight. Plates were either sealed and stored at 4°C or used directly. Plates were stable for a minimum of 6 months without loss of activity.

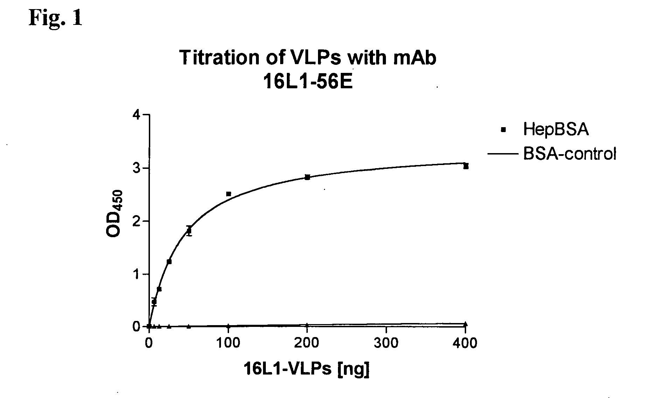

The amounts of VLPs of HPV-16, -18, -33, and -39 indicated in the figure legend of Fig. 1 were added to individual wells in a total volume of 100 µl PBS-0.05% Tween20 and incubated for 1 h at room temperature. As control served a BSA-coated plate. Plates were washed three times with PBS-0.05% Tween20 and subsequently incubated for 1 h at 37°C with the indicated VLP-specific antibodies. After washing, plates were incubated with a horseradish peroxidase-coupled secondary antibody for 30 min at 37°C. The plates were intensively washed and incubated with TMB (KPN) for 10 min at 37°C. The reaction was stopped with 1N HCI after 10 min. Optical density was measured in a Multiscan Microtiter Reader EX at 450 nm. Figure 1 clearly demonstrates that VLPs of all types tested bind efficiently to heparin-BSA-coated microtiter plates whereas only background binding was observed to control plates. Saturation was reached with approximately 400 ng of VLPs for all HPV-types tested.

### Example 2 (Kinetics of VLP binding)

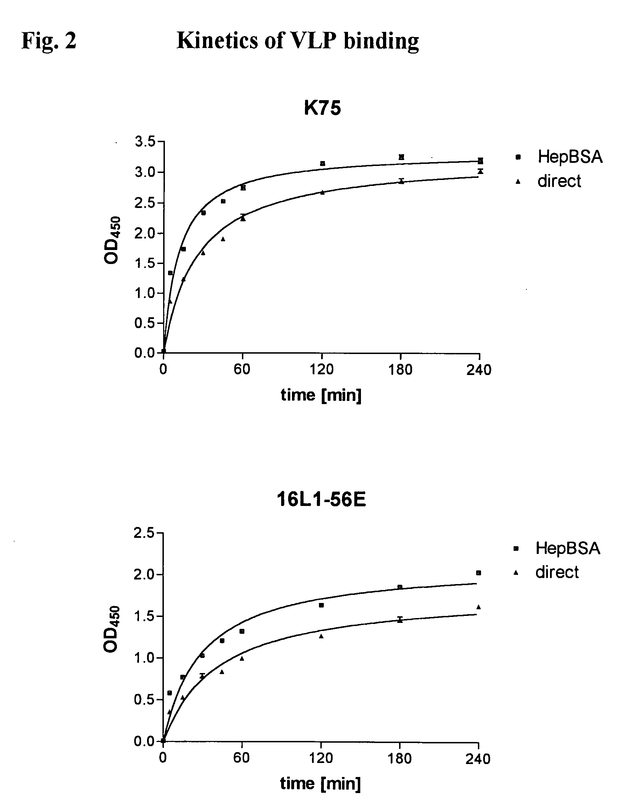

Fig. 2 delineates that the kinetic of VLP binding to heparin-BSA-coated plates is faster than direct binding of VLPs to the plate. 200 ng of HPV-16 VLPs were incubated in a time course with either heparin-BSA-coated plates or with untreated MaxiSorb plates and ELISA was performed as described above using either HPV-16 VLP-specific polyclonal antibody K75 or the conformation-dependent and virus-neutralizing monoclonal antibody (mAb) 16L1-56E. Whereas saturation was reached after 2 hrs using heparin-BSA-coated plates it took 4 hrs to reach saturation when VLPs were coupled directly to microtiter plates. Antiserum K75 and 16L1-56E yielded similar results. This demonstrates that saturation of VLP binding to heparin-BSA coated plates is significantly faster than to the plastic surface of the microtiter plate.

### Example 3 (Binding of properly folded and denatured L1 protein to heparin)

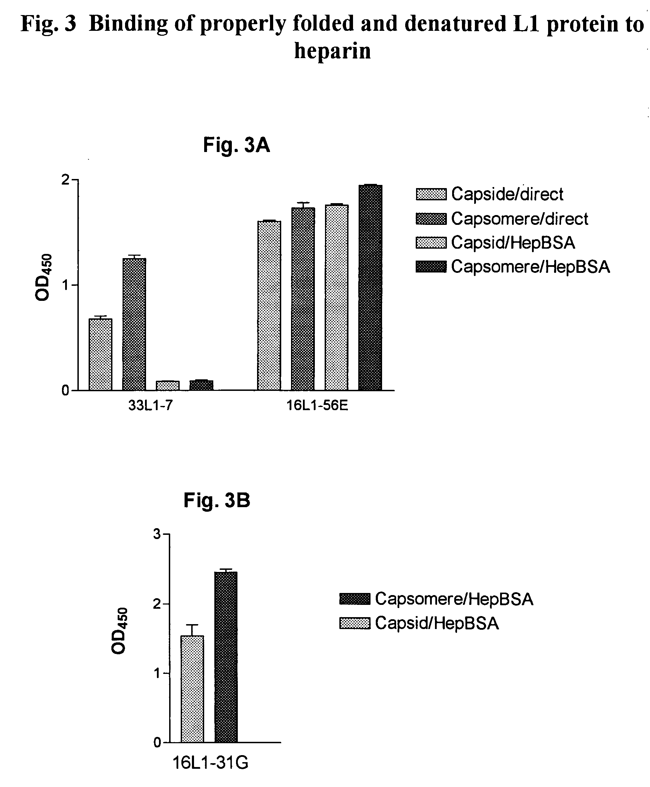

VLPs prepared in heterologous expression systems always contain a fraction of denatured incorrectly folded L1 protein in addition to the L1 protein assembled to capsomeres or VLPs. The detection of heparin-bound VLPs with the conformation-dependent monoclonal antibody 16L1-56E already suggested that correctly folded L1 protein bound to heparin. To test if denatured L1 protein can also attach to heparin we used monoclonal antibodies that recognize linear epitopes hidden in the VLP and capsomere structure (Sapp et al. 1994) for detection of heparin-bound VLPs. We used microtiter plates to which HPV-16 VLPs were coupled directly as controls. As shown in Fig. 3a, monoclonal antibody 33L1-7 efficiently bound to VLP-coated plates. The reactivity was increased significantly when the VLPs were denatured prior to coating as expected (Sapp et al. 1994). In contrast, no reactivity was found when mAb 33L1-7 was incubated with VLPs that were coupled to plates via heparin. Identical results were obtained with VLPs from other HPV-types and with additional mAbs binding to epitopes hidden in capsomeres and VLPs (not shown). Monoclonal antibody 16L1-56e served as control, showing that equivalent amounts of VLPs bound to the microtiter plate.

We also used mAb 16L1-31G that recognizes an epitope, which is hidden inside of the VLP structure but accessible to antibody binding in monomeric capsomeres. This antibody definitely detected VLPs coupled indirectly via heparin. The reactivity was increased when capsomeres instead of VLPs were used (Fig. 3b). Capsomeres were generated by reduction of intercapsomeric disulfide bonds as described (Sapp et al, 1995, 1998). This clearly demonstrates that VLPs and capsomeres bind to heparin, whereas denatured L1 protein does not bind to heparin-BSA coated plates.

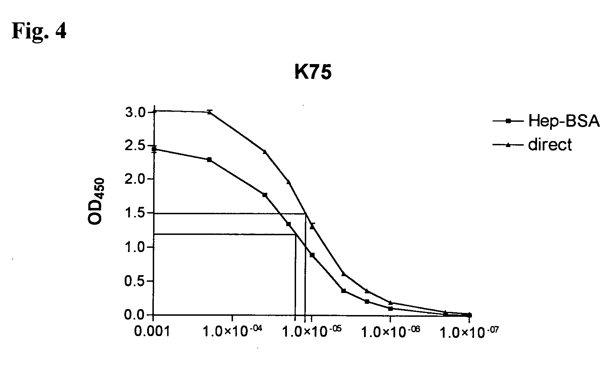

These observations were confirmed when we determined the titers of polyclonal antibodies raised in rabbits or mice against VLPs. Since VLPs are contaminated with denatured L1 protein and proteins derived from the host cell used for generation of VLPs, we expect an immune response to be also directed against these contaminations. This was evident because titers determined by ELISA were always significantly higher than neutralization titers, which were determined using an in vitro neutralization assay (Unckell et al., 1997; Fligge et al., 2001). We therefore compared the antibody titers of such antisera obtained with VLPs directly coated to microtiter plates or bound indirectly using heparin-BSA. The mean geometric antibody titers determined with heparin-BSA-coated microtiter plates were 2 fold lower than the titers obtained with ELISA plates to which VLPs were coupled directly (Fig. 4). This again suggests that heparin selectively binds L1 protein assembled to capsomeres or VLPs. We assume that contaminations derived from the host cell will also not bind to heparin.

### Example 4 : (Detection of HPV 16 specific antibodies in the serum of randomly selected blood donors and patient treated for cervical carcinoma.)

MaxiSorb microtiter plates (Nunc) were coated with 100 µl/well of 1 µg/ml Heparin-BSA (heparin covalently attached to bovine serum albumin; Sigma Aldrich) dissolved in phosphate buffered saline (PBS) overnight at 4°C. Plates were washed with PBS-0.05% Tween20 three times and subsequently incubated with blocking solution (PBS-0.05% Tween20-0.5% BSA) for 3 h at room temperature. Plates were washed again with PBS and H₂O and subsequently dried under vacuum overnight. Plates were either sealed and stored at 4°C or used directly.

Each well was incubated with 100 ng of VLPs of HPV-16 in a total volume of 100 µl PBS-0.05% Tween20 and incubated for 1 h at room temperature. As control served wells incubated with 100 µl PBS-0.05% Tween20 without VLPs. Plates were washed three times with PBS-0.05% Tween20 and subsequently incubated for 1 h at 37°C with a 1:25 dilution of human sera from patients treated for cervical carcinoma or from sera randomly selected from blood donors. After washing, plates were incubated with a horseradish peroxidase-coupled secondary antibody for 30 min at 37°C. The plates were intensively washed and incubated with TMB (KPN) for 10 min at 37°C. The reaction was stopped with 1N HCI after 10 min. Optical density was measured in a Multiscan Microtiter Reader EX at 450 nm.

Results were obtained by subtracting the optical densities of the wells serving as negative control from the VLP 16 coated wells.

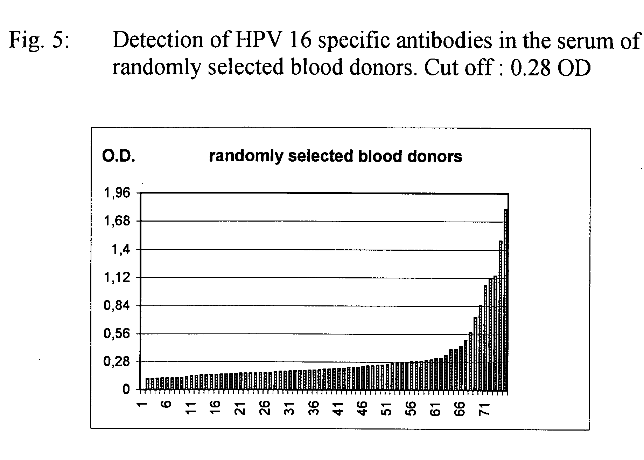

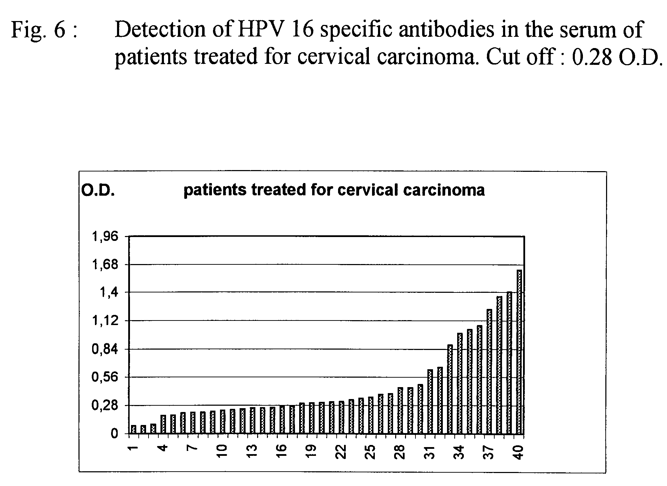

Figures 5 and 6 clearly demonstrates that the described assay is suitable to detect HPV 16 specific antibodies in the serum of the indicated groups. Around 20% of the sera of randomly selected blood donors (Figure 5), but over 50% of the sera of patient treated because of a cervical carcinoma (Figure 6) are positive for HPV 16 specific antibodies.

## Claims

1. Assay for the detection of HPV type-specific antibodies comprising
a) a substrate coated with at least one negatively charged macromolecule, and
b) at least one type of virus-like particles or subunits of these virus like particles containing at least one HPV type-restricted epitope.

2. Assay according to claim 1, wherein the substrate is selected from the group consisting of microplates and membranes.

3. Assay according to any one of the preceding claims, wherein the negatively charged macromolecule is a negatively charged polysaccharide selected from the group consisting homo- and heteroglycans containing COO⁻ and/or SO₃⁻ -groups.

4. Assay according to claim 3, wherein the negatively charged polysaccharide is a sulphated-glucosaminoglycan.

5. Assay according to claim 4, wherein the sulphated-glucosaminoglycan is Heparin.

6. Assay according to any one of the preceding claims, wherein the at least one type of virus-like particles or subunits of these virus-like particles containing at least one HPV type-restricted epitope is at least one virus-like particle of HPV 6, 11, 16, 18, 31, 33, 35, 39, 45, 56 and/or 58.

7. Assay according to claim 6, wherein one type of virus-like particles selected from the group, consisting of virus-like particles of HPV 16, 18, 33 and/or 39 is present.

8. Assay according to any one of the preceding claims, wherein the virus-like particles contain as the at least one HPV-type restricted epitope at least one capsid protein selected from the group consisting of L1 and L2 of HPV or mixtures thereof.

9. Assay according to claim 8, wherein the HPV type-restricted epitope is the capsid protein L1.

10. Use of an assay according to any one of the preceding claims in the detection of HPV type-specific antibodies.

11. Substrate, coated with a negatively charged macromolecule.

12. Substrate according to claim 11, wherein the negatively charged macromolecule is a negatively charged polysaccharide.

13. Substrate of claim 12, wherein the negatively charged polysaccharide is Heparin.

14. Process for the detection of HPV type-specific antibodies comprising the steps of
a) providing an assay according to claims 1 to 9,
b) contacting the assay with a probe, and
c) detecting whether an HPV type-specific antibody is bound to the assay.

15. Process according to claim 14, wherein the probe is selected from the group consisting of human serum, blood and any other liquid containing antibodies.

16. Process according to any one of claims 14 or 15, wherein the detection is carried out by using an anti-human immunglobulin and any appropriate detection system.'

17. Process according to any one of claims 14 to 16, wherein the HPV type-specific antibody is selected from the group consisting of HPV 6, 11, 16, 18, 31, 33, 35, 39, 45, 56 and/or 58 antibody.
